# Europäisches Patentamt

## European Patent Office

## Office européen des brevets

(19)

(11) **EP 1 032 337 B1**

(12) **EUROPEAN PATENT SPECIFICATION**

(45) Date of publication and mention
of the grant of the patent:
**27.10.2004   Bulletin 2004/44**

(21) Application number: **97953032.6**

(22) Date of filing: **13.11.1997**

(51) Int Cl.⁷: **A61F 13/15**

(86) International application number:
**PCT/US1997/020792**

(87) International publication number:
**WO 1999/025284 (27.05.1999 Gazette 1999/21)**

(54) **ABSORBENT ARTICLES WITH MEANS FOR ACHIEVING OR MAINTAINING CONVEXO-CONCAVE BUNCHING**

ABSORBIERENDE ARTIKEL MIT MITTELN, UM KONVEX-KONKAVE WÖLBUNGEN ZU ERREICHEN UND BEIZUBEHALTEN

ARTICLE ABSORBANT COMPORTANT DES MOYENS PERMETTANT DE DONNER OU MAINTENIR UNE CONFIGURATION CONVEXE-CONCAVE

(84) Designated Contracting States:
**AT BE CH DE DK ES FI FR GB GR IE IT LI LU NL PT SE**

(43) Date of publication of application:
**06.09.2000   Bulletin 2000/36**

(73) Proprietor: **THE PROCTER & GAMBLE COMPANY Cincinnati, Ohio 45202 (US)**

(72) Inventors:
  • **WILLMS, Eric, Joachim**
    **D-65812 Bad Soden (DE)**
  • **SCHMITT, Achim**
    **D-55424 Münster-Sarmsheim (DE)**
  • **WOSCHNIK, Thomas**
    **D-53881 Euskirchen (DE)**

  • **MICCIO, Silvio**
    **D-53123 Bonn (DE)**
  • **THURNAY, Eva, Susanne**
    **D-76344 Eggenstein-Leopoldshafen (DE)**
  • **BLANCO, Agustin, Ramos**
    **D-61440 Oberursel (DE)**

(74) Representative: **McGregor, Judit Ester Procter & Gamble Service GmbH Sulzbacher Strasse 40-50 65924 Schwalbach am Taunus (DE)**

(56) References cited:
**EP-A- 0 585 904          WO-A-93/01781**
**WO-A-96/41602          US-A- 2 064 431**
**US-A- 5 127 911          US-A- 5 611 790**

EP 1 032 337 B1

**Description**

General field of the invention

The present invention relates to hygienic absorbent articles, in particular to absorbent articles designed for receiving larger amounts of body fluids as urine and / or fecal material, such as adult incontinence articles or baby diapers.

Background / Prior art

Absorbent articles for use in hygienic applications are well known in the art. Also, the shaping of such absorbent articles has been discussed in depth, both with regard to contouring of the article (in contrast to e.g. rectangular), but also with regard to three dimensional shape.

In particular feminine hygiene pads have been disclosed having improved body contact for receiving the body exudates by threedimensionally shaping the article.

US-A-5.300.055 (Buell) discloses an absorbent article having a body facing surface which has a convex upward surface, such that the total article either takes the shape of an "inverted U" (i.e. the opening of the U facing away from the wearer) or of a "W". In WO 95/31165 (Olsen) a similar article is described, aiming at a "W" shaped configuration by means of a resilient component as an integral part of the article.

In WO 95/17148 (Bergman), sanitary napkins are disclosed, which achieve an improved contact between the labia of the wearer and the article for improved fluid (i.e. menses) handling capability. Also WO 88/04547 (Thoren) describes a sanitary napkin aiming at improved contact between the article and the wearer by attaching elastic members both in MD and in CD direction to that side of the topsheet which is oriented away from the wearer. Also EP-A-0.302.523 (Lassen) describes an anatomically - shaped feminine pad, made by forming, moulding or other forming techniques. WO 93/01781 describes sanitary napkins with close body contact throughout their length.

In US-A-5.098.4233 (Pieniak) fit aspects of absorbent articles such as baby diapers are discussed. The primary focus is put against narrow crotch widths, such as by folding the diaper in the crotch region 2x, 4x, 6x, or 8x. Such folding could be achieved by so called W-folding, whereby the absorbent article takes the shape of a "W". There is no disclosure about the shaping or folding of the core in relation to the chassis elements.

However, prior art has not sufficiently considered, what the specific requirements and benefits of shaping in the context of adult incontinence articles or baby diapers with relatively high amounts of fluid discharges are.

Also, the prior art failed to achieve further benefits of specific shaping for articles intended for receiving faecal material.

Hence, there is still a need to improve both the comfort aspects of such articles and the handling of the body exudates at the same time.

Objects of the invention

Hence it is an object of the present invention to provide absorbent articles particularly designed for receiving relatively large amounts of body exudates such as urine, whereby the articles do not raise the discomfort for the wearer unduly, without detrimentally impacting on performance of the article.

It is a further aspect of the present invention, to provide such articles having additionally improved handling capability with regard to feces.

It is a further object of the present invention to provide designs which at the same time satisfy these requirements and allow easy application to the wearer, either by him- or herself, or by another person such as a parent or a caretaker.

Summary

The present invention relates to absorbent articles comprising a means to achieve or maintain a convexo-concave shape with an upwardly bulged (i.e. towards the wearer during use) center crotch part, and outwardly bulged parts at the longitudinal ends of the article following the body curvature to surround the waist.

Brief description of drawings

Figure 1 shows an absorbent pad as an example of an absorbent element;

Figure 2a shows the cross-sectional view of a thin slice of the urinary loading zone of an article bunched in the Ω-shape according to the present invention;

Figure 2b shows the cross-sectional view of a thin slice of the urinary loading zone of a comparative prior art article in a W-fold shape.

Detailed description

An absorbent article generally comprises:

- an absorbent member (often referred to as core or core structure, which may consist of sub-structures);
- "chassis elements", such as
- a fluid impervious backsheet;
- optionally further features like closure elements
- or elastification.

Within the context of the present description, an absorbent article can be "unitary", i.e. all elements are adjoined together such that the article is - when being used - essentially one piece, or the absorbent article can consist of separate elements, such as having an absorbent element and an external fixation element, e.g. a re-usable fixation stretch garment such as often practiced for the adult incontinence articles in form of a elasticized pant combination with an absorbent disposable pad.

In either case, the article or its elements can be disposable or reusable, whereby the term "disposable" is used herein to describe absorbent articles or elements which are not intended to be laundered or otherwise restored or reused as an absorbent article or element (i.e. they are intended to be discarded after a single use and, preferably, to be recycled, composted or otherwise disposed of in an environmentally compatible manner).

The absorbent article has a lateral direction (x direction or width) defined as the direction parallel to the lateral centerline and being aligned with a "left-to-right" direction of the wearer when being used; the longitudinal direction (y direction or length) being defined as the direction parallel to the longitudinal centerline and being aligned with the height direction of a wearer in a standing position during use; and the axial direction (Z direction or thickness) being defined as the direction extending through the thickness of the diaper 20.

The term "concave" means - in the context of the present invention - a curvature which is "outwardly bulged", such as when following the general curvature of the waist of a human wearer, i.e. a belt worn around the waist would exhibit a generally concave curvature. The term "convex" relates to the opposite curvature. Of course, these terms are relative, and also linked to terms "upwards" and "downwards", which follow the general understanding of gravity. Henceforth, a letter "U" would has a "downward convex", or a concave, shape. The Greek capital letter Ω (Omega) has in the center part an "upwardly convex" curvature. If the terms "concave" and "convex" are used without an "upward' or "downward" direction, the meanings is as with "upward". If these terms are used in referring to a wearers position, this is assumed, unless otherwise noted, that the wearer is in a standing position.

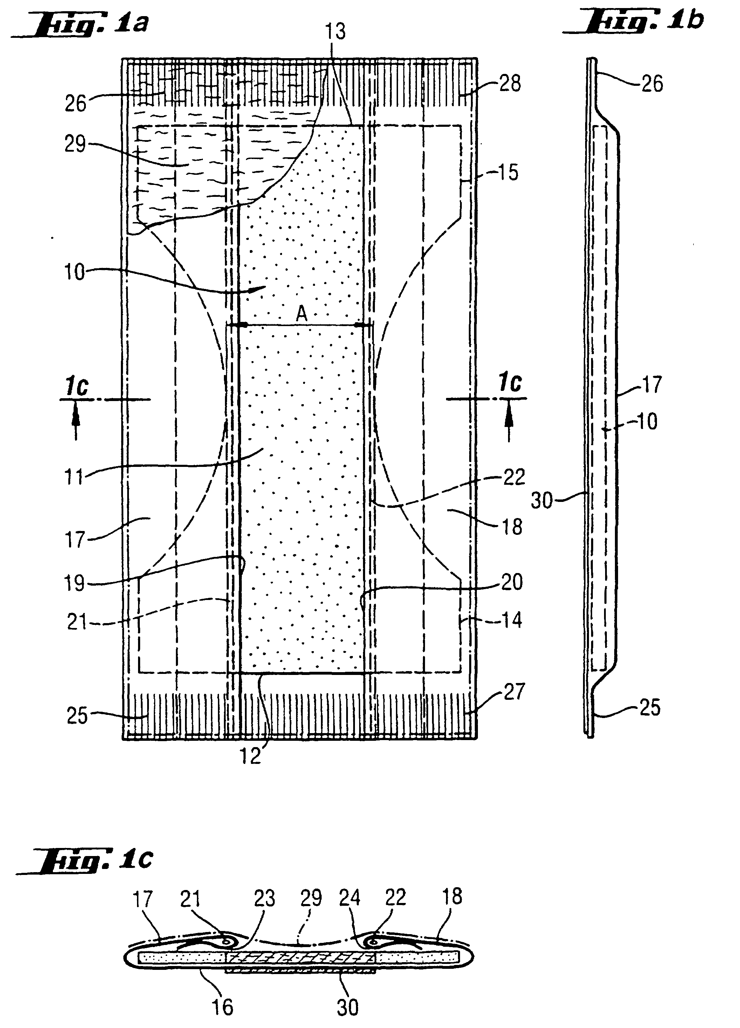

In Fig. 1 a, b, and c the present invention is exemplified by describing the absorbent element of an absorbent article, which could be a baby diaper or an adult incontinence pad.

The pad comprises an absorbent core 10, designed for absorbing and containing fluids, in most instances primarily aqueous based. The absorbent core 10 may be any absorbent means which is generally compressible, conformable, and capable of absorbing and retaining primarily aqueous liquids. Examples of suitable absorbent materials include comminuted wood pulp, creped cellulose wadding; meltblown polymers; chemically stiffened, modified or cross-linked cellulosic fibres; tissue including tissue wraps and tissue laminates; absorbent foams; absorbent sponges; superabsorbent polymers; absorbent gelling materials; or any equivalent material or combinations of materials. The configuration and construction of the absorbent pad may also be varied (e.g., the absorbent pad may have varying caliper zones, a hydrophilicity gradient, a superabsorbent gradient, or lower average density and lower average basis weight acquisition zones; or may comprise one or more layers or sub-structures). Preferably the absorbent pad has an essentially flat configuration so as to avoid complications which could arise from having a strongly threedimensionally shaped form.

Exemplary absorbent structures for use in the absorbent pad as used in the disposable industry are described in U.S. Patent 4,610,678 entitled "High-Density Absorbent Structures" issued to Weisman et al. on September 9, 1986; U.S. Patent 4,673,402 entitled "Absorbent Articles With Dual-Layered Cores" issued to Weisman et al. on June 16, 1987; U.S. Patent 4,888,231 entitled "Absorbent Core Having A Dusting Layer" issued to Angstadt on December 19, 1989; and U.S. Patent 4,834,735, entitled "High Density Absorbent Members Having Lower Density and Lower Basis Weight Acquisition Zones", issued to Alemany et al. on May 30, 1989.

US patent no 4,411,660 discloses in an absorbent product two layers of absorbent material of different types, such that the upper layer gels slower than the first layer.

European Patent Specification EP-B-0 401 189 discloses that favorable properties of absorbent products can be achieved by using two different types of absorbent gelling material in separate layers, rather than as a mixture of the two absorbent gelling materials in a single layer.

The hydrogel-forming absorbent polymers useful in the present invention include a variety of substantially water-insoluble, but water-swellable polymers capable of absorbing large quantities of liquids. Such polymers materials are also commonly referred to as "hydrocolloids", or "superabsorbent" materials. These hydrogel-forming absorbent polymers preferably have a multiplicity of anionic, functional groups, such as sulfonic acid, and more typically carboxy, groups. Examples of polymers suitable for use herein include those which are prepared from polymerizable, unsaturated, acid-containirig monomers. Thus, such monomers include the olefinically unsaturated acids and anhydrides that contain at least one carbon to carbon olefinic double bond. More specifically, these monomers can be selected from olefinically unsaturated carboxylic acids and acid anhydrides, olefinically unsaturated sulfonic acids, and mixtures thereof.

As described above, the hydrogel-forming absorbent polymers are preferably slightly network crosslinked. Network crosslinking serves to render the polymer substantially water-insoluble and, in part, determines the absorptive capacity and extractable polymer content characteristics of the precursor particles and the resultant macrostructures. Processes for network crosslinking the polymers and typical network crosslinking agents are described in greater detail in the herein before-referenced U.S. Patent 4,076,663, and in DE-A-4020780 (Dahmen).

In order to be able to compare absorbent articles for varying end use conditions, or differently sized articles, the "design capacity" has been found to be a suitable measure.

For example, babies are representing a typical usage group, but even within this group the amount of urine loading, frequency of loading, composition of the urine will vary widely from smaller babies (new-born babies) to toddlers on one side, but also for example among various individual toddlers.

Another user group may be larger children, still suffering from a certain form of incontinence.

Also, incontinent adults can use such articles, again with a wide range of loading conditions, generally referred to as light incontinence ranging up to severe incontinence.

Henceforth, absorbent articles being able to cope with such requirements should have the capability of picking up such amounts of urine, which will be referred to for the further discussion as "design capacity", which is descibed in more detail below.

These amounts of fluids have to be absorbed by materials which can ultimately store the bodily fluids, or at least the aqueous parts of these, such that - if any - only little fluid is left on the surface of the article towards the wearers skin. The term "ultimate" refers in one respect to the situation as in the absorbent article at long wearing times, in the other respect to absorbent materials which reach their "ultimate" capacity when being equilibrated with their environment. This can be in such an absorbent article under real in-use conditions after long wearing times, or this also can be in a test procedure for pure materials or material composites. As many of the processes under consideration have asymptotic kinetic behavior, one skilled in the art will readily consider "ultimate" capacities to be reached when the actual capacity has reached a value sufficiently close to the asymptotic endpoint, e.g. relative to the equipment measurement accuracy.

As an absorbent article can comprise materials which are primarily designed to ultimately store fluids, and other materials which are primarily designed to fulfill other functions such as acquisition and/or distribution of the fluid, but may still have a certain ultimate storage capability, suitable core materials according to the present invention are described without attempting to artificially separate such functions. Nonetheless, the ultimate storage capacity can be determined for the total absorbent core, for regions thereof, for absorbent structures, or even sub-structures, but also for materials as being used in any of the previous.

As discussed in the above for varying the dimensions of the article, one skilled in the art will be able to readily adopt the appropriate design capacities for other intended user groups. For example, for Adult Incontinence articles intended for use with severely incontinent persons can contain 9 g of superabsorbent material having an absorbent capacity of about 31 ml/g when submitted to the well known Teabag centrifuge capacity test, and contain 97 g of conventional cellulosic airfelt having a capacity of about 4 ml/g, thus resulting in a total ultimate storage capacity of about 667 ml. Other examples relate to articles for light incontinent persons. For example ATTENDS MINI has an ultimate storage capacity of about 70 ml, ATTENDS MINI PLUS of 90 ml, or ATTENDS NORMAL of about 167 ml, with all these products sold by Procter & Gamble in various countries in Europe.

Referring again to Figures 1, said absorbent core has waist regions 14, 15 next to the longitudinal edges 12, 13, and a crotch region 11 connecting these regions 14, 15, which has a minimum width ÒA". As exemplified in Fig. 1a, the width in the waist region 14, 15 is about twice the width of the crotch region 11.

The absorbent core 10 is connected with a fluid impervious backsheet 16. The backsheet 16 may comprise a woven or nonwoven material, polymeric films such as thermoplastic films of polyethylene or polypropylene, or composite materials such as a film-coated nonwoven material. Often, the backsheet is a thermoplastic film having a thickness of from about 0.012 mm (0.5 mil) to about 0.051 mm (2.0 mils), such as a blown or cast PE film as available under the designation RR8220 (blown films) and RR5475 (cast films) as manufactured by Tredegar Industries, Inc. of Terre Haute, IN. Such a backsheet 16 is preferably embossed and/or matte finished to provide a more clothlike appearance.

Further, and often preferably, the backsheet 16 may permit vapours to pass through while still preventing liquids from penetrating through the backsheet 16.

Backsheet 16 may have both width and length dimensions exceeding the dimensions of the absorbent core 10, thereby forming peripheral edges. The region of the backsheet 16 which extend outwardly of the core in longitudinal direction is referred to as endflaps.

The relatively wide lateral edges 17, 18 of the backsheet 16 are folded inwardly so as to at least partially overlaying the absorbent core 10. The free longitudinal edges 19, 20 comprise an elastification feature 21, 22, such as an elastic band or stripe. When the absorbent article is stretched out flat as indicated in Fig. 1 these elastic features are stretched so as to allow contraction for better body conformity during use.

The lateral edges 17, 18 are now in a tubular arrangement 23, 24 with the unfolded parts, whereby the stretched elastics which are connected at their ends with the edges 25, 26 respectively 27, 28 of the inwardly folded lateral edges 17, 18.

A topsheet 29 is positioned on the absorbent core 10 oriented towards the wearer during use. The topsheet 29 is compliant, soft feeling. Further, the topsheet 29 is liquid pervious permitting fluids like urine liquids to readily penetrate through its thickness. A suitable topsheet may be manufactured from a wide range of materials, such as porous foams; reticulated foams; apertured plastic films; or woven or nonwoven webs of natural fibres (e.g., wood or cotton fibres), synthetic fibres (e.g., polyester or polypropylene fibres), or a combination of natural and synthetic fibres. There are a number of manufacturing techniques which may be used to manufacture the topsheet 29. For example, the topsheet 29 may be a nonwoven web of fibres spunbonded, carded, wet-laid, meltblown, hydroentangled, combinations of the above, or the like. An often used topsheet is carded and thermally bonded by means well known to those skilled in the fabrics art, for example made of staple length polypropylene fibres such as is manufactured by Veratec, Inc., a Division of International Paper Company, of Walpole, Massachusetts under the designation P-8.

The topsheet may be essentially attached throughout most or all of the surface of the absorbent core, or it may be only partially bonded thereto. The topsheet may comprise apertures to ease penetration of exudates there through, such as urine or feaces. The topsheet may further be fully or partially elasticated.

In order to keep the absorbent element as described in the above on the body of the wearer and in registry with the body fluid releasing body openings, the absorbent article has to have one or more fixation means.

Such a fixation means can of the mechanical type, i.e. essentially aiming at overcoming the gravitational forces and / or the frictional forces between the article and outside elements, such as the outer clothing of the wearer and /or the bedding elements by exploiting the frictional forces between the article and the skin of the wearer, or structural support forces such as registry with body contours such as hip bones of the wearer.

The absorbent element may alternatively be hold in place by conventional closure systems to fix the article or the absorbent element around the waist of the wearer, such as by integral tape systems, which can comprise adhesive coated tapes or mechanically engaging elements.

Thereby, the absorbent article or the absorbent element is provided with a closure system comprising a tape tab fastening device and a landing member. The tape tab fastening device comprises an attachment area and a functional area, and the landing member is preferably a reinforcing strip or the alternative a portion of the backsheet. The functional elements may take on a number of configurations such as adhesive fastening elements, mechanical fastening elements, a combination of adhesive fastening elements and mechanical fastening elements, or any other means as are known to the man skilled in the art.

For these closure systems which encircle the waist of the wearer, the article is preferably further comprising elastification features operatively connected with these closure feature, so as to enhance and sustain the fit of the article during use.

Absorbent articles, which are sometimes referred to as "pant-style diapers" are unitary articles with a pant style design - i.e. with closed side seams to form a fully closed structure in the waist region - together with elastification features to allow fixation as the mentioned elastic pant, and also an absorbent core such as a panty comprising elasticized region.

Alternatively, an absorbent element such as described in the above, may simply have a means for attaching it to the normal underwear of the user, such as often applied for light feminine hygiene articles, such as so-called "panty liner", and indicated in Fig. 1c by fixation means 30.

An even further alternative has a fastening means of the topical adhesive attachment type. Such "body adhesive" means aim at fixation of the article directly to the skin of the wearer.

A further embodiment of the present invention relates to non-unitary articles, comprising an absorbent element and a separate fixation means or garment such as a net or stretch pant, which can be used either as replacement or in addition to the regular underwear of the wearer. A similar approach to such non-unitary elements can be an attachable belt as described in EP-A-0.409.307 (Gipson et al.)

Further, absorbent articles according to the present invention can have several of the above mentioned fixation means, such as having both a body adhesive applied to the absorbent element, and an additional stretch or garment in a dual fixation means arrangement.

In case of a non-unitary arrangement, both the absorbent element and the fixation element can be disposable, or one of these or both can be reusable, such as after washing.

$\Omega$- (Omega)-shaped absorbent articles

In addition to the features as described above, the following embodiments make the present invention particularly suited for being used to receive urinary and / or fecal exudates.

The first and essential element is a feature to provide a convex bulging at least in the crotch region, and in particular at least in the urine loading region or zone, during application of the article to the wearer and / or during use. The urine loading zone is generally located somewhat, i.e. often about 5 cm, forward from the crotch point of the article, which corresponds to the narrowest width dimension of the stiffest part of the article.

This convex bulging can be achieved by means arranged in the crotch region of the article and in particular in the urinary loading zone of the article, with positioning the center line of the article during use in closer proximity to the body of the wearer than the lateral side edges of the absorbent member closer together than in a flat shape and thus have its center line positioned closer towards the wearer$\overline{O}$s body than the side edges, such that the absorbent member is in fact upwardly bulging towards the wearer.

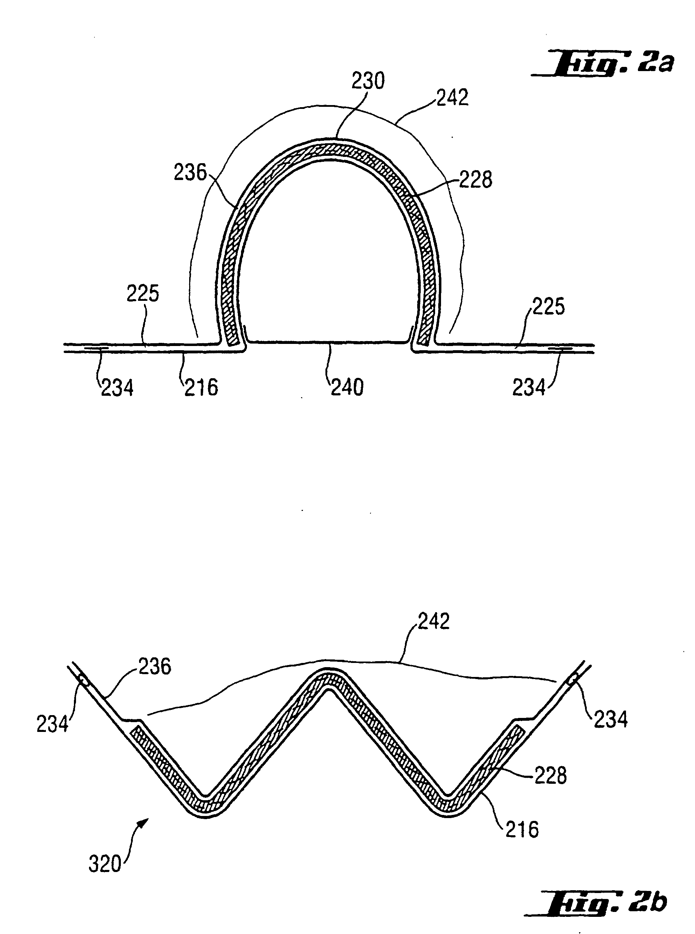

In Fig. 2a, such an absorbent core 228 is forming the upwardly convex shape (i.e. the center part of an $\Omega$ (Omega). The lower parts of the $\Omega$ (Omega) are formed by chassis elements, i.e. primarily backsheet materials such as films and/or nonwovens. This configuration is referred to as "$\Omega$-bunching".

This convex $\Omega$-shape of the absorbent core has to be at least around the urinary loading point, preferably extending at least about +/- 2.5 cm to the front and towards the back of the article. In order to allow comfortable wearing, this convex shaping of the crotch region core will transition into a concavely shaped curvature of the front and rear waist parts of the article to fit well around the waist of the wearer.

The elastic elements to keep the side margins of the article in contact with the wearers legs are also positioned with the zones of the lower part of the $\Omega$.

This provides particular benefits versus prior art articles adopting the shape of a "U", or the shape of a W as depicted in Fig. 2b, schematically showing a cross-section through a thin slice of a prior art article 320, with the total absorbent element shaped or folded in this W-form.

The $\Omega$- (Omega)-bunching of the present invention allows to form pockets of longitudinally shape outside of the absorbent member, which can provide certain benefits during use. As the absorbent member will be loaded with at its top "ridge" (230), it will on one side catch the body exudates effectively, as there is only little room for the fluids to go elsewhere. Second, gravity aids - even when high performance materials and structures are used - the penetration of the fluid into the receiving material, thereby minimizing the residence time of the liquid on the surface, and thus the potential for wetting the skin of the wearer or for leaking to the outside.

This advantage becomes even more pronounced, when being combined with core structures having a sub-optimal liquid handling performance. Even if the urine would not penetrate into the article immediately upon contact with the article, it would "run off" along the slopes of the upwardly oriented convex bulge (i.e. the slopes of the $\Omega$ [Omega]). If - as might occur with cores of lower performance or capacity - the core is (permanently or momentarily) saturated in this wetted region, fluid that would not penetrate into the core, will be caught by the impermeable sides of the $\Omega$ (Omega), which provide good sealing against the outer clothing of the wearer by the liquid impermeable backsheet (216).

A further advantage becomes apparent in figures 2a and 2b, wherein the absorbent core is depicted after it has absorbed significant amounts of urine. This leads to imminent swelling of the core as indicated by the borders after swelling 240 and 242, which, in case of a conventional "W-folded" core, will fill the deeper parts of the W, almost creating a completely filled cup - in particular under the impact of movement of the wearer. In particular for cores comprising superabsorbent materials, which tend to reduce liquid permeability of the matrices they are comprised in, this can lead to the situation where the lower parts of the W (oriented away from the wearer), are hindered in their swelling - be this by geometrical expansion constraint or by lack of fluid transport to these parts.

In case of the present invention, however, the core will swell only the convex bulging of the "$\Omega$", without substantial impeding negatively fluid transport .

If in combination with a high performing core, the functionality of the $\Omega$-bunching with regard to fluid handling changes: now the requirement for the lateral seal becomes less important, but the "$\Omega$-ridge" (230) provides CD-directional flow, which however, will increase the liquid acquisition rate by increasing the loading area in CD-direction, thus also enabling significantly improved longitudinal fluid distribution.

Further, when the article is loaded with feces, these can be deposited into these longitudinally oriented pockets positioned laterally outwardly of the absorbent core, thereby leaving the surface of the absorbent member free for receiving further urinary loading. In particular with relatively thin (or runny, or lower viscosity) feces, these can - e.g. when the wearer is in a standing position due to gravity - flow further down into the crotch region. However, having an upward bulge there, and non-absorbent side sealing, the feces will not cover the absorbent core and not deteriorate its functionality as to receiving urine.

In both instances, the backsheet (216) has to withstand such loading, and must not - even when designed to allow vapors to penetrate through - allow permeation under normal in-use conditions.

As it will also be readily appreciated, these longitudinal side pockets need to be sealed against the skin of the wearer, such as by elastic means (234), positioned between the backsheet (216) and the topsheet (236).

For the scope of the present invention, such pockets may also be formed by so called "leg-cuffs" as well known in the art such as for baby diaper, e.g. in EP-A-0.263.720. In a preferred embodiment of such "leg cuffs", the upstanding part of the secondary cuff is positioned laterally outwardly from the fixed base part of the same cuff, such that it is avoided that these cuffs align on the surface of the absorbent core, and thus reduce the ability to quickly receive even repeated gushes well.

A particularly preferred embodiment for the present invention comprises a chassis whereby these pockets are made by inwardly folding and attaching the backsheet and topsheet material having elastic strands at its longitudinally running edges, such as described in EP-A-0.098.512 (Beckestrøm).

A further element of the present invention results form the transition of the convex bulging in the crotch zone to a concave bulging in the waist zone when being worn.

As is well known from static considerations, arc-like structures have a certain structural strength. This structural strength is an essential element of the present invention in the crotch region, whereby the upward bulging creates such an arc-like structure. As indicated in the above, this convex bulging has to be transformed into a concave structure in the waist region of the article. As can be readily visualized with piece of paper which is folded in one direction in the middle and into the opposite direction at either end, this transition zone has a significant strength and resistance to deformation. In the current context, this results in "pockets" being formed - or void spaces between the article and the body of the wearer - during use. Such pockets can have a desired effect, as feces can be caught in void spaced formed by the $\Omega$-bunching on the wearer, namely where the convex shape in the crotch region transforms into the concave shape in the rear upper waist part of the article, creating a "rear feces pocket". Such pocket can have a volume suitable for receiving fecal discharges, and comprise from about 10cm$^3$ to about 500cm$^3$ or more.

Such pockets also can have a benefit in a gender specific article design, namely when such a pocket is formed by the front transition zone, thereby forming a space for male genitals.

Such pockets may also be undesired, for example in articles intended for light incontinence use without intention to be loaded with feces, or in the front zone of a genderized female usage product, especially with absorbent cores being relatively stiff by themselves (such as through highly densified cellulosic structures)

In all cases, it will be desirable to have the transition not at an arbitrary position, but rather at the desired location. Henceforth, a preferred embodiment of the present invention includes "hinges" for defined transitioning from the convex to the concave shape.

The relative dimensions of the convex, concave and transition regions depends of course on the intended use and wearer. However, generally human body configurations are such that the lateral extension of the article during use should be small, preferably less than 7 cm, more preferably less than 5 cm, and most preferably even less than about 4 cm. In case of a W-folded core, this dimension relates 4 times the core thickness, in case of the $\Omega$-bunched core, this relates to only 2 times the core thickness (upon neglecting the thickness of the non-core related materials). The length of the convexly bunched region should be at least about 5 cm, to allow adequate contact in the discharge region. The upper limit for this region depends on the intended use, and ranges from only little more than the minimum of 5 cm as describe (e.g. for female, urinary incontinence products) to about 20 cm for articles intended to also receive feces and requiring an "rear feces pocket".

After having described how the various elements of the present invention interact with each other, a more detailed description of the individual features will follow.

<u>Means for sustained $\Omega$ (Omega)-shape</u>

At present, many absorbent articles are produced flat, i.e. having no specific three-dimensional shape. The person who applies the article (i.e. the user him- or herself, or a helping person such as caretaker, parent or the like) can apply the article in various shapes, and by various application steps.

Even if absorbent articles are applied to the wearer such that they form the preferred $\Omega$-shape at least in the crotch zone, normal in-use movements or the loading itself can result in the $\Omega$-shape not being maintained sufficiently pronounced and / or for a sufficiently long period. Thus, in order to maintain the $\Omega$-shape, the article is provided with a means for enhancing maintenance of the Ω-shape during application of the article to the wearer, and also during the use period.

Such a means to provide Ω-bunching can be any means, which is able to deform or to maintain the deformation of the absorbent member in the crotch region such that the above describe shape is formed during application and during use

The most simple way to achieve the Ω-shape during application of the article to the wearer is the folding of the article in the appropriate shape by the manufacturer. Therefore, the article needs to be folded along the longitudinally extending centerline such that the topsheet, which is intended to be oriented to the wearer during use, lies outwardly and the backsheet, which is intended to lie outwardly or towards the clothing of the wearer inwardly of this folded part, which must comprise at least the crotch region, and especially the loading region. With this folding, the person applying the article has a means to readily achieve the Ω-bunched shape upon application.

Alternatively, the article can be folded in any way by the manufacturer, but it comprises a means which just prior to application creates the Ω-shape or supports the Ω-shape when manually created by the person applying the article.

Such means can be elastic features, which - when being in a non-Ω-shape configuration - are stretched, and which contract upon application, thereby forming the Ω-shape, or it can be means for affixing respective backsheets parts to each other.

One way to achieve this is by simply applying adhesive means in a conventional manner similar to the adhesives applied in feminine hygiene products as so called "panty fastening adhesives" for affixing the article to the panty of the wearer. In the present case, however, the objective is to attach backsheet parts not to wearers clothing, but together.

The adhesive can be applied during the manufacturing and then be covered by release papers as well known for the "panty fastening adhesives". The release paper will then be removed at the time of application, just before the respective parts of the backsheet are brought together to form the Ω-shaping. It becomes apparent, that in the first case, packing and shipping of the article is easier, whilst in the second case care must be taken so as to not damage / deteriorate the shaping during transport.

Alternatively, the Ω-shape can be created during manufacturing of the article. In this case, such an adhesive means can be applied to the article when being flat and in an unbulged shape, and the respective parts of the backsheet are then brought together so as to create the bunching.

A further alternative for such an Ω-bunching means is to use mechanical attachment means, such as generally referred to as "mechanical fastening means", whereby a first member being applied to one part (e.g. made of hooks) is mechanically engaging in a second member (e.g. a looped landing zone).

An even preferred alternative for affixing the respective parts of the backsheet together is by not doing so in a firm way but by allowing some relative movement of the backsheet surfaces vs. each other in the longitudinal direction. This relative movement provides particular benefits during the movement of the wearer, such as when walking. Then, the Ω-shaped tunnel will be somewhat "distorted" without, however loosing its functionality nor its general Ω-shape.

This is particularly useful, if the articles are intended to be worn by mobile persons, such as mobile, non-bed-ridden adults, or toddlers, as it will allow increased comfort during walking.

Such a feature can be one or more elastic bands or stripes (such as indicated with 240 in Fig. 2a) having at least a CD-directional contractional force component. They can be attached towards the lateral edges (i.e. towards the waist regions) of the backsheet but not to the central part (i.e. in the crotch region) of the backsheet, thus pulling together the lateral edges, thus forming the upward bulge or Ω-bunch. Whilst the elastic means for the present invention not necessarily has to be affixed at the outer edges (i.e. the lower base of the "Ω"), care must be taken to shape the article such that only the upper convex part of the Ω is formed by the absorbent core and not a complete W shape is taken, thereby (i.e. to have no absorbent core in the region of the side flaps 225). This can be achieved by the core having a certain stiffness in combination with the structural stiffness resulting from the bunching.

Such elastic elements can be positioned outside the backsheet, or inside, they should however be positioned underneath the absorbent core member (i.e. directed away from the wearer), as otherwise the risk of resulting in a U-shape configuration is too high.

Application of CD-directional stretch features has been disclosed for example in EP-A-0.652.175.

Another preferred alternative can be to position the fixation means in some distance from the backsheet such as by a spacer or block element, thereby forming a hinge between the left and right side of the lateral longitudinal edges of the article.

In another preferred embodiment, not only the backsheet parts are brought into close contact, but also to further bond the total absorbent member through its entire thickness.

This allows that the shape will be maintained better even under stressful in-use conditions.

This bond should, whilst being sufficiently strong to withstand in use stress and also wetting, be sufficiently soft to not increase the discomfort of the wearer.

Other ways to achieve bonds between the backsheets, between backsheet and additonal means for maintaining and sustaining Ω-bunching are other well known techniques such as glue application, melt-bonding, and the like.

A particularly preferred execution in the context of a air-permeable backsheet such as a nonwoven material is the application of pointwise application of hot air thereby melt-bonding certain parts of the structure together.

In a further execution of the present invention, the upwardly oriented convex shaping (i.e. the Ω-shape) of an absorbent member can be sustained by a separate means which creates an upwardly oriented force in the crotch region (i.e. towards the wearer during use).

In a first execution, such a lifting element can be integrated into a unitary article, such as by positioning elastic elements, which have a contractional force component in the longitudinal direction of the article, on the side of the absorbent member, which is during use oriented away from the wearer. Thereby, the elements should be at least in the crotch and/ or loading zone be in the longitudinal centre line zone. i.e. be not too far away from the longitudinal centre line to form the Ω-bulge, which generally can be achieved if these are not less than 2.5 cm offset the centre line in either / or left and right direction.

In a second embodiment, such a lifting element can be in a separate element, which is not integral or unitary with the element of the absorbent core (i.e. the absorbent pad). Such means can be a separate garment, such as a net pant, to be worn over the absorbent article. A particularly preferred garment for such an application is described in co-filed Patent Application, Attorney Docket, CM1637Q.

Such a suitable garment comprises an elasticized waistband, a front panel having first and second sections, a rear panel having first and second sections, a crotch region disposed between and joining the front panel to the rear panel and a pair of elasticized leg openings. The first section of the front panel has a greater resistance to stretching in the lateral direction than the second section of the front panel. The first section of the rear panel has a greater resistance to stretching in the lateral direction than the second section of the rear panel. The crotch region is provided with a longitudinal stretch control member that is disposed along the longitudinal centerline of the undergarment. The longitudinal stretch control member limits the stretch of the crotch region in the longitudinal direction causing the crotch region to conform to a wearer's skin surface. A front stretch control member is disposed in the front panel and extends from the longitudinal stretch control member to the waistband. A rear stretch control member is disposed in the rear panel and extends from the longitudinal stretch control member to the waistband.

While such a suitable garment can be assembled from materials that may be known to the art as having the requisite mechanical properties, it is preferably knit, such that the mechanical properties of the various components thereof can be provided by a combination of the knit pattern used for a particular component and the yams that are used. In a particularly preferred embodiment, the longitudinal stretch control member is integrally knit with the crotch region, the front stretch control member is integrally knit with the front panel, and the rear stretch control member is integrally knit with the rear panel.

Further, two or more of the above elements may be combined in the absorbent article. For example, a pre-folded absorbent pad can be held in place by means of the preferred stretchable garment, as described in the above. Or, a non-prefolded pad can be brought into the Ω-shape configuration by an elastic which is in an extended state during shipment, and upon application creates the Ω-shape configuration, and this pad can be held in place by a conventional stretchable garment, such as a pant, without a specific feature for sustaining the Ω-shape, or by a improved Ω-shape supporting garment. Also, the combination of body adhesives with a pre-folded unitary article comprising MD oriented elastification means for providing a lifting force on the crotch or loading zone can be suitable.

Hinge lines

As has been explained in the above, the "convexo-concave" transition should not be positioned arbitrarily, but should happen at a desired place. This can be achieved by including a "hinge means" in the article, and in particular in the absorbent core, if this is the stiffest element of the article.

Without this additional means, this transition will generally occur somewhat outside the crotch region, both in the front and back region, where due to the absence of the restraining legs of the wearer, the Ω-shape can flatten, and thus allows somewhat easier transition, which still might result in undesired deformation or weakening of the structure.

Thus, it becomes clear, that it will be highly beneficial to define the transition region from concave bulging to convex bulging, which can be achieved by creating hinge lines within the absorbent structure.

Such hinge lines can be created by various means, such as cutting, or embossing (i.e. creating high density lines throughout the absorbent, such that the bending at the edges of these high density lines is eased), or low density lines (e.g. created by forming the article with regions having a lower basis weight of materials, but maintaining the same caliper).

The hinge lines to enhance the convexo-concave transition must extend from the outer longitudinal edges of the core or the stiffness determining element of the article into the direction of the longitudinal center line, i.e. the hinge lines have to be arranged such that they have a cross-directional orientation. These hinge lines can be straight lines or curved ones thereby allowing optimization of the specific design of the article, and the anatomy of the intended wearer group. Also the length of the hinge lines can be readily adopted. Also, several independent lines can be used within each one of the transition zones, or one line can split into other. Preferably, such hinge lines do not run through the full width of the absorbent member, as this might result in an undesired loss in strength, or integrity, in particular during use.

Such hinge lines can be created by various means, such as cutting, or embossing (i.e. creating high density lines throughout the stiff member such as the absorbent core, thereby easing the bending at the edges of these high density lines), or low density lines (e.g. as achieved by creating lower basis weight zones of material in the stiff element and densifying it to a lesser degree than the surrounding higher basis weight regions).

Design Capacity and Ultimate Storage Capacity

In order to be able to compare absorbent articles for varying end use conditions, or differently sized articles, the "design capacity" has been found to be a suitable measure.

For example, babies are representing a typical usage group, but even within this group the amount of urine loading, frequency of loading, composition of the urine will vary widely from smaller babies (new-born babies) to toddlers on one side, but also for example among various individual toddlers.

Another user group may be larger children, still suffering from a certain form of incontinence.

Also, incontinent adults can use such articles, again with a wide range of loading conditions, generally referred to as light incontinence ranging up to severe incontinence.

Henceforth, such articles being able to cope with such requirements should have the capability of picking up such amounts of urine, which will be referred to for the further discussion as "design capacity".

These amounts of fluids have to be absorbed by materials which can ultimately store the bodily fluids, or at least the aqueous parts of these, such that - if any - only little fluid is left on the surface of the article towards the wearers skin. The term "ultimate" refers in one respect to the situation as in the absorbent article at long wearing times, in the other respect to absorbent materials which reach their "ultimate" capacity when being equilibrated with their environment. This can be in such an absorbent article under real in-use conditions after long wearing times, or this also can be in a test procedure for pure materials or material composites. If the processes under consideration have asymptotic kinetic behavior, one skilled in the art will readily consider "ultimate" capacities to be reached when the actual capacity has reached a value sufficiently close to the asymptotic endpoint, e.g. relative to the equipment measurement accuracy.

As an absorbent article can comprise materials which are primarily designed to ultimately store fluids, and other materials which are primarily designed to fulfill other functions such as acquisition and/or distribution of the fluid, but may still have a certain ultimate storage capability, suitable core materials according to the present invention are described without attempting to artificially separate such functions. Nonetheless, the ultimate storage capacity can be determined for the total absorbent core, for regions thereof, for absorbent structures, or even sub-structures, but also for materials as being used in any of the previous.

In case of applying the present invention to other articles requiring different end-uses, one skilled in the art will be able to readily adopt the appropriate design capacities for other intended user groups.

In order to determine or evaluate the Ultimate Design Storage Capacity of an absorbent article, a number of methods have been proposed.

In the context of the present invention, it is assumed, that the Ultimate Storage Capacity of an article is the sum of the ultimate absorbent capacities of the individual elements or material. For these individual components, various well established techniques can be applied as long as these are applied consistently throughout the comparison. For example, the Tea Bag Centrifuge Capacity as developed and well established for superabsorbent polymers can be used for such materials, but also for others (see above).

Once the capacities for the individual materials are known, the total article capacity can be calculated by multiplying these values (in ml/g) with the weight of the material used in the article.

For materials having a dedicated functionality other than ultimate storage of fluids - such as acquisition layers and the like - the ultimate storage capacity can be neglected, either as such materials do in fact have only very low capacity values compared to the dedicated ultimate fluid storage materials, or as such materials are intended to not be loaded with fluid, and thus should release their fluid to the other ultimate storage materials.

With such definitions, for example a so-called "panty liner" product exhibits very low Ultimate storage capacities of a few ml or less. Feminine Hygiene pads have often up to about 20 ml, light urinary incontinence articles have for example 75 ml or about 90ml, medium urinary incontinence articles, or also smaller baby diaper can have about 165 ml, and toddler size baby diapers reaching 300 ml or more, and severe adult incontinence article having 600 ml or more of ultimate storage capacity.

Teabag Centrifuge Capacity Test

The Teabag Centrifuge Capacity test measures the Teabag Centrifuge Capacity values, which are a measure of the retention of liquids in the gelling material at hydrostatic pressure.

The superabsorbent material is placed within a "teabag", immersed in a 0.9 % by weight sodium chloride solution for 20 minutes, and then centrifuged for 3 minutes. The ratio of the retained liquid weight to the initial weight of the dry superabsorbent material is the absorptive capacity of the superabsorbent material.

21 of 0.9% by weight sodium chloride in distilled water is poured into a tray having dimensions 24cm x 30 cm x 5cm. The liquid filling height should be about 3cm.

The teabag pouch has dimensions 6.5cm x 6.5cm and is available from a company called Teekanne in DYsseldorf, Germany. The pouch is heat sealable with a standard kitchen plastic bag sealing device (e.g. VACUPACK$_2$ PLUS from Krups, Germany).

The teabag is opened by carefully cutting it partially, and is then weighed. A 0.200g +/- 0.005g sample of the superabsorbent material is placed in the teabag. The teabag is then closed with a heat sealer. This is called the sample teabag.
An empty teabag is sealed and used as a blank.

Each teabag is then held horizontally, and the sample teabag is shaken so as to distribute the superabsorbent material evenly throughout the bag. The sample teabag and the blank teabag are then laid on the surface of the saline solution, and submerged for about 5 seconds using a spatula to allow complete wetting (the teabags will float on the surface of the saline solution but are completely wetted). The timer is started immediately.

After 20 minutes soaking time the sample teabag and the blank teabag are removed from the saline solution, and placed in a Bauknecht WS130, Bosch 772 NZK096 or equivalent centrifuge (230 mm diameter), so that each bag sticks to the outer wall of the centrifuge basket. The centrifuge lid is closed, the centrifuge is started, and the speed increased quickly to 1,400rpm. Once the centrifuge has been stabilized at 1,400rpm the timer is started. After 3 minutes, the centrifuge is stopped.

The sample teabag and the blank teabag are removed and weighed separately.

The Teabag Centrifuge Capacity (TCC) for the sample of superabsorbent hydrogel-forming material is calculated as follows:

$$TCC = [(\text{sample teabag weight after centrifuging}) - (\text{blank teabag weight after centrifuging}) - (\text{dry superabsorbent hydrogel-forming material weight})]$$

$$\div (\text{dry superabsorbent material weight}).$$

**Claims**

1. Disposable absorbent diaper for receiving urine and feces,
comprising a topsheet (29), a backsheet (16) and a core (10) arranged therein between,
wherein said core (10) comprises a front and a rear end region, which both are concavely shaped to conform with the body of the wearer, being concave regions,
**characterised in that** said core (10) comprises an urinary loading zone, having a convex bulging shape oriented upwardly towards the wearer, being a convex zone,
wherein said convex bulging shape is achieved by means of a prefold of the diaper along the longitudinal centreline of the diaper at least in said urinary loading zone such that at least parts of the backsheet (16) covering the absorbent core (10) are in direct contact with each other,
and wherein said diaper further comprises elasticated longitudinal cuffs (21, 22) formed by said backsheet (16) or by a separate liquid impervious cuff material,
and **in that** the diaper comprises (a) transition region(s) between the concave region(s) and the convex zone, whereby said transition region(s) of the absorbent core (10) form(s) at least a pocket located longitudinally offset towards the rear.

2. Absorbent diaper according to claim 1, wherein said convex bulging shape of the urinary loading zone is achieved by means of an adhesive applied outwardly on the backsheet (16).

3. Absorbent diaper according to claim 1, wherein said convex bulging shape of the urinary loading zone is achieved by means of a mechanically engaging means at least a part of which being positioned laterally spaced away from the longitudinally extending center line of the diaper.

4. Absorbent diaper according to any of claims 1 to 3 wherin said convex bulging shape of the urinary loading zone is achieved by an elastically contractible element (234) with at least a CD-directional force component.

5. Absorbent diaper according to any of claims 2 to 4 wherein said means for achieving the convex bulging shape of the urinary loading zone is affixed to the lateral sides of the core (10, 228), and being essentially unaffixed in the center.

6. Absorbent diaper according to any of claims 1 to 5, having a length and a width dimension, and a z-direction perpendicular to both these dimensions, wherein said means for achieving the convex bulging shape of the urinary loading zone is an elastically contractible element (234) with at least an MD-directional force component at least exerting a z-directional force component oriented towards the wearer during use.

7. Absorbent diaper according to any of claims 1 to 6 wherein the diaper further comprises fixation means for fixation of the diaper on the body of the wearer.

8. Absorbent diaper according to claim 7 wherein said fixation means comprise tapes and landing zones which adhesively or mechanically affix the front and back end regions of the diaper to each other.

9. Absorbent diaper according to claim 7 wherein said fixation means further comprise elastic members to maintain the diaper in position.

10. Absorbent diaper according to any of the preceding claims, wherein the absorbent core (10) has a design capacity of more than 70 ml urine, preferably more than 105 ml or even more than 300 ml.

**Patentansprüche**

1. Absorbierende Wegwerfwindel zur Aufnahme von Urin und Fäkalien, umfassend eine Decklage (29), eine Außenlage (16) und einen dazwischen angeordneten Kern (10),
wobei der Kern (10) einen vorderen und einen hinteren Endbereich aufweist, welche beide konkav geformt sind, um sich nach dem Körper des Trägers zu richten, die konkave Bereiche sind,
**dadurch gekennzeichnet, dass** der Kern (10) eine Urin-Empfangs-Zone aufweist, die eine konvexe ausbauchende Form aufweist, die in Richtung auf den Träger nach oben gerichtet ist, die eine konvexe Zone ist,
wobei die konvexe ausbauchende Form durch eine Vorfaltung der Windel entlang der longitudinalen Mittellinie der Windel mindestens in der Urin-Empfangs-Zone erreicht wird, sodass mindestens Abschnitte der Außenlage (16), die den absorbierenden Kern (10) bedecken, im direkten Kontakt miteinander stehen,
und wobei die Windel ferner elastische longitudinale Bündchen (21, 22) umfasst, die durch die Außenlage (16) oder ein separates flüssigkeitsundurchlässiges Bündchenmaterial gebildet sind,
und dass die Windel (einen) Übergangsbereich(e) zwischen dem/n konkaven Bereich(en) und der konvexen Zone umfasst,
wobei der/die Übergangsbereich/e des absorbierenden Kerns (10) mindestens eine Tasche bilden(t), die longitudinal versetzt in Richtung auf den hinteren vorgesehen ist.

2. Absorbierende Windel nach Anspruch 1, wobei die konvexe ausbauchende Form der Urin-Empfangs-Zone durch einen Klebstoff erreicht wird, der außen auf die Außenlage (16) aufgebracht ist.

3. Absorbierende Windel nach Anspruch 1, wobei die konvexe ausbauchende Form der Urin-Empfangs-Zone durch ein mechanisch eingreifendes Mittel erreicht wird, wobei mindestens ein Abschnitt von diesem seitlich beabstandet zu der longitudinal verlaufenden Mittellinie der Windel vorgesehen ist.

4. Absorbierende Windel nach einem der Ansprüche 1 bis 3, wobei die konvexe ausbauchende Form der Urin-Empfangs-Zone durch ein elastisch zusammenziehbares Element (234) mit mindestens einer CDdirektionalen Kraft-Komponente erreicht wird.

5. Absorbierende Windel nach einem der Ansprüche 2 bis 4, wobei das Mittel zum Erreichen der konvexen ausbauchenden Form der Urin-Empfangs-Zone an den lateralen Seiten des Kerns (10, 228) befestigt ist und im Wesentlichen im Zentrum unbefestigt ist.

**6.** Absorbierende Windel nach einem der Ansprüche 1 bis 5, mit einer Längen- und einer Breiten-Abmessung und einer zu diesen beiden Abmessungen senkrechten z-Richtung, wobei das Mittel zum Erreichen der konvexen ausbauchenden Form der Urin-Empfangs-Zone ein elastisch zusammenziehbares Element (234) mit mindestens einer MD-direktionalen Kraft-Komponente ist, die mindestens eine z-direktionale, in Richtung auf den Träger während des Gebrauchs gerichtete Kraft-Komponente ausübt.

**7.** Absorbierende Windel nach einem der Ansprüche 1 bis 6, wobei die Windel ferner Anbringmittel zum Anbringen der Windel an dem Körper des Trägers umfasst.

**8.** Absorbierende Windel nach Anspruch 7, wobei die Anbringmittel Bänder und Aufnahme-Bereiche umfassen, welche adhäsiv oder mechanisch die vorderen und hinteren Endbereiche der Windel aneinander befestigen.

**9.** Absorbierende Windel nach Anspruch 7, wobei die Anbringmittel ferner elastische Elemente zum Halten der Windel in Position umfassen.

**10.** Absorbierende Windel nach einem der vorherigen Ansprüche, wobei der absorbierende Kern (10) ein Auslegungs-Fassungsvermögen von mehr als 70 ml Urin, vorzugsweise von mehr als 105 ml oder sogar von mehr als 300 ml aufweist.

**Revendications**

**1.** Couche absorbante jetable, destinée à recevoir de l'urine et des matières fécales,
comprenant une feuille de dessus (29), une feuille de fond (16), et une âme (10) placée entre elles,
où ladite âme (10) comprend une région d'extrémité avant et une région d'extrémité arrière, qui sont toutes les deux façonnées en une forme concave de façon à épouser la forme du corps de l'utilisateur, dites régions concaves,
**caractérisée en ce que** ladite âme (10) comprend une zone de chargement urinaire, ayant une forme protubérante convexe, orientée vers le haut, en direction de l'utilisateur, dite zone convexe,
où ladite forme protubérante convexe est obtenue au moyen d'un pliage préalable de la couche le long de la ligne médiane longitudinale de la couche, au moins dans ladite zone de chargement urinaire, de sorte qu'au moins les parties de la feuille de fond (16) qui recouvrent l'âme absorbante (10) soient en contact direct l'une avec l'autre,
et où ladite couche comprend, en outre, des revers longitudinaux élastifiés (21, 22) formés par ladite feuille de fond (16) ou par un matériau de revers distinct, imperméable aux liquides,
et **en ce que** la couche comprend (a) une ou des régions de transition entre la ou les régions concaves et la zone convexe,
la ou lesdites régions de transition de l'âme absorbante (10) formant ainsi au moins une poche située en une position décalée vers l'arrière dans la direction longitudinale.

**2.** Couche absorbante selon la revendication 1, dans laquelle ladite forme protubérante convexe de la zone de chargement urinaire est obtenue au moyen d'un adhésif appliqué à l'extérieur sur la feuille de fond (16).

**3.** Couche absorbante selon la revendication 1, dans laquelle ladite forme protubérante convexe de la zone de chargement urinaire est obtenue à l'aide de moyens entrant mécaniquement en prise, dont au moins une partie se trouve dans une position distante, en direction latérale, de la ligne médiane longitudinale de la couche.

**4.** Couche absorbante selon l'une quelconque des revendications 1 à 3, dans laquelle ladite forme protubérante convexe de la zone de chargement urinaire est obtenue au moyen d'un élément (234) pouvant se contracter de manière élastique, ayant au moins une composante de force dans la direction CD.

**5.** Couche absorbante selon l'une quelconque des revendications 2 à 4, dans laquelle lesdits moyens pour obtenir ladite forme protubérante convexe de la zone de chargement urinaire sont fixés aux côtés latéraux de l'âme (10, 228), en n'étant pratiquement pas fixés au centre.

**6.** Couche absorbante selon l'une quelconque des revendications 1 à 5, ayant une certaine dimension en longueur et en largeur, et une direction z perpendiculaire à ces deux dimensions, où lesdits moyens pour obtenir ladite forme protubérante convexe de la zone de chargement urinaire sont formés d'un élément (234) pouvant se contracter de manière élastique, ayant au moins une composante de force dans la direction MD, qui exerce au moins une composante de force dans la direction z, orientée vers l'utilisateur pendant l'utilisation.

**7.** Couche absorbante selon l'une quelconque des revendications 1 à 6, dans laquelle la couche comprend, en outre, des moyens de fixation pour fixer la couche sur le corps de l'utilisateur.

**8.** Couche absorbante selon la revendication 7, dans laquelle lesdits moyens de fixation comprennent des rubans et des zones réceptrices, qui fixent les régions d'extrémité avant et arrière de la couche l'une à l'autre, de manière adhésive ou mécanique.

**9.** Couche absorbante selon la revendication 7, dans laquelle lesdits moyens de fixation comprennent, en outre, des éléments élastiques destinés à maintenir la couche en position.

**10.** Couche absorbante selon l'une quelconque des revendications précédentes, dans laquelle l'âme absorbante (10) a une capacité nominale de plus de 70 ml d'urine, de préférence, de plus de 165 ml, ou même, de plus de 300 ml.

**Fig. 1a**

**Fig. 1b**

**Fig. 1c**

*Fig. 2a*

*Fig. 2b*